# EUROPEAN PATENT SPECIFICATION

(11) **EP 2 303 297 B1**
(45) Date of publication and mention of the grant of the patent: **30.05.2012**
(21) Application number: 09757609.4
(22) Date of filing: 05.06.2009
(51) Int. Cl.: A23L 1/29, A61K 36/282, A23L 2/52, A61P 3/02, A61P 3/08, A61P 21/06, A23L 1/30

(54) **WATER-SOLUBLE EXTRACTS OF ARTEMISIA DRACUNCULUS (TARRAGON) FOR IMPROVEMENT OF GLUCOSE METABOLISM**
WASSERLÖSLICHE EXTRAKTE VON ARTEMISIA DRACUNCULUS (ESTRAGON) ZUR VERBESSERUNG DES GLUCOSESTOFFWECHSELS
EXTRAITS SOLUBLES DANS L'EAU D'ARTEMISIA DRACUNCULUS (ESTRAGON RUSSE) UTILISÉS POUR L'AMÉLIORATION DU MÉTABOLISME DU GLUCOSE

(30) Priority: 06.06.2008 EP 08157794; 06.06.2008 US 131140 P
(43) Date of publication of application: 06.04.2011
(73) Proprietor: Finzelberg GmbH & Co. KG, 56626 Andernach (DE)
(72) Inventor: PISCHEL, Ivo, 53547 Rossbach (DE); WALBROEL, Bernd, 53639 Königswinter (DE); FEISTEL, Bjoern, 56626 Andernach (DE)
(74) Representative: von Kreisler Selting Werner
(86) International application number: PCT/EP2009/056920
(87) International publication number: WO 2009/147228

(56) References cited:
- WO-A-2006/010560
- US-A1- 2003 072 822
- ANONYMOUS: "Estragon vrac 30 g" WIKIPEDIA, [Online] 20 January 2008 (2008-01-20), XP002503316 Retrieved from the Internet: URL:http://web.archive.org/web/20080120195 252/http://www.naturalia.fr/bio-estragon-v rac-30-g_2267_6_525.htm> [retrieved on 2008-11-10]
- ANONYMOUS: "Estragonessig" WIKIPEDIA, [Online] 11 October 2007 (2007-10-11), XP002503317 Retrieved from the Internet: URL:http://web.archive.org/web/20071011230 806/http://www.rezeptewiki.org/wiki/Estrag onessig> [retrieved on 2008-11-10]
- DATABASE WPI Week 200404 Thomson Scientific, London, GB; AN 2004-039516 XP002503321 & JP 2003 339366 A (ARIAKE JAPAN KK) 2 December 2003 (2003-12-02)
- ANONYMOUS: "Estragon" WIKIPEDIA, [Online] 25 October 2007 (2007-10-25), XP002503318 Retrieved from the Internet: URL:http://web.archive.org/web/20071025041 935/http://fr.wikipedia.org/wiki/Estragon> [retrieved on 2008-11-10]
- ANONYMOUS: "Essig" WIKIPEDIA, [Online] 24 December 2005 (2005-12-24), XP002503319 Retrieved from the Internet: URL:http://web.archive.org/web/20051224235 658/http://de.wikipedia.org/wiki/Essig> [retrieved on 2008-11-10]
- ANONYMOUS: "Deutscher Estragon" WIKIPEDIA, [Online] 12 September 2008 (2008-09-12), XP002503320 Retrieved from the Internet: URL:http://de.wikipedia.org/wiki/Deutscher _Estragon> [retrieved on 2008-11-10]

## Description

The present invention relates to water-soluble extracts derived from Russian Tarragon, their preparation, fractionation and their use, either alone or as combinations with further compounds.

The modern civilization especially of the western world is threatened by a number of diseases related to the rising prosperity. Thus, the incidences of obesity, cardio-vascular and metabolic diseases, like metabolic syndrome, increased blood lipids and diabetes type II are increasing dramatically. Especially, the ingestion of high amounts of simple sugars may lead to high blood concentrations of glucose and thus, after a longer period of time, to glucose intolerance, metabolic syndrome and often finally to diabetes type II. Very critical in this context are the high glucose peaks that occur post-prandial, which can lead to glycoxylation reactions in the blood and certain tissues, probably followed by secondary diseases.

Additionally, the lack of physical workout and intake of diets characterized by high fat intake and repeated ingestion of refined foods and sugars, coupled with low fiber and vegetable intake, along with the natural aging process, causes a deterioration in the way in which the body metabolizes blood glucose. When the body cannot properly metabolize blood glucose, a tendency to store glucose as fat typically occurs. This is one reason levels of body fat increase with age. On the other hand constantly high blood concentrations of glucose or high post-prandial glucose peaks can be related to undesirable glycoxylation reactions of blood or tissue components. Secondarily, a glucose tolerance and/or insulin resistance can be developed, which may be related to metabolic syndrome and diabetes type 2. Diabetes is also known to be associated with a variety of other ailments including heart disease, hypertension, and obesity. There is a known link between insulin resistance and increased visceral adiposity. Diabetes is also a leading cause of glaucoma and other conditions related to a decrease in the quality of life.

The object of this invention was thus to provide an extract, useful for normalizing elevated blood glucose levels under conditions of metabolic syndrome, pre-diabetes and diabetes Type 2 in animals and humans. Therefore, the aim for this invention is to prevent or counteract, and treat the general deterioration of the health status while aging or under the condition of illnesses and/or diseases related to high blood glucose levels.

The object is solved by an extract, obtainable by
- extracting Russian Tarragon with water or a mixture of water with up to 20% by volume of a water-miscible solvent selected from C1 to C3 alcohols.

Surprisingly, the extracts of the invention show improved antidiabetic properties compared to extracts of prior art.

According to the invention, the application of the disclosed Tarragon extracts and/or combinations of the said extracts with excipients, supplements, nutrients and alike will assist to normalize elevated blood glucose levels under conditions of metabolic syndrome, pre-diabetes and diabetes Type 2 in animals and humans.

Surprisingly, it was found that the oral administration of the aqueous Russian tarragon extracts showed a change of blood glucose levels within a well established animal model using a glucose tolerance test, which is well transferable to human post-prandial lowering of blood glucose that occurs after meals. The results are clearly improved over the formerly described and existing, state-of-the-art products containing ethanolic extracts of Tarragon.

Disclosed herein are inter alia orally applicable products comprising an water-soluble extract of Russian Tarragon, fractions, or derivatives thereof without or with formulation constituents and a method of lowering or normalize elevated blood glucose levels under conditions of metabolic syndrome, pre-diabetes and diabetes Type 2 in animals and humans comprising administration of the said products as dietary supplement, food or drink preparation, or in a pharmaceutical delivery form.

Furthermore, it is an embodiment of this invention to provide a method and an orally applicable product or combination which will prevent, counteract and/or treat conditions of metabolic syndrome, pre-diabetes and diabetes Type 2 as well as support weight loss and body fat reduction in animals and humans.

US 6,893,627 discloses Tarragon (Artemisia dracunculus) and plant extracts of tarragon are considered as food and generally recognized as safe (GRAS) and can be administered orally to humans or animals for the purpose of controlling blood glucose. It discloses an 60 % ethanolic plant extract of freeze-dried fresh plants derived from hydroponically grown Tarragon. Prior to this patent publication a few scientific papers described the traditional use of tarragon for diabetes type 2, although they do not differentiate between French and Russian Tarragon and the kind of preparation was not described either (e.g. Swantson-Flatt SK, Day C, Flatt PR, Gould BJ, Baily C7. Glycemic effects of traditional plants treatments for diabetes: studies in normal and streptozocin induced diabetic mice. Diabetes Res 1989; 34(2): 132-135).

The aqueous Russian Tarragon extract of the present invention helps to clear glucose from the blood, reduces post-prandial glucose peaks and helps reducing undesired glycoxylation reaction with vital molecular body structures or metabolites in the blood serum or tissues.

Furthermore the invention present very convenient forms of Russian Tarragon extracts, which enables many technical feasible applications, due to its outstanding properties, like complete water solubility that allow all imaginable beverage formulations. The extraction process is less expensive compared to solvent extraction, because water can be used as exclusive or predominant extraction media. The manufacturing process is much easier designed as for a solvent extraction, e.g. with higher ethanol concentrations above 50% V/V. An ethanolic or solvent extraction bears the disadvantages that lipophilic compounds are extracted in higher concentrations than using water as extraction media. Thus, the prior art extracts from solvent extraction are more of pharmaceutical character and efficacy, and additionally it is not completely water-soluble and not applicable in drinks and beverages and many other dietary supplement or food products.

The extraction is preferably conducted at a temperature range of 20 to 95, preferably 50 to 80°C.

A suitable extraction time is between 1 hour to 40 hours, preferably 5 hours to 20 hours.

The extraction medium is water.

In addition, the disclosed water extract of Russian Tarragon can be obtained completely free of the supposing perilous essential oils, like estragol and methlyeugenol. In addition, it was found that the disclosed extracts do not have an effect on the basal blood glucose levels, a fact which can be interpreted as crucial positive safety aspect related to missing of a hypoglycemia status after oral administration of the said extract by fasting individuals.

The different botanical basic extracts were obtained by standard laboratory methods but were additionally transferable and could be up-scaled to technical production. Furthermore, fractionations were obtained by using membrane filtration and absorption column. Commercially available Russian Tarragon can be used as raw material for an extraction with water or hydroethanolic extraction solvent. The raw materials were cultivated on open fields, harvested and gently dried in a conventional drier commonly used for drying herbs and spices.

Suitable extracts can for example be produced as follows: 1 kg of the raw materials is extracted twice with 8 L water at 80°C or 50°C, respectively. After cooling of the eluates over night, the solutions are filtered through paper filters and the solvent is evaporated by means of a rotatory evaporator. The obtained dense extracts, were mixed with 30% of suitable carrier, like maltodextrins, hydrolysed collagen, microcrystalline cellulose or cellulose derivatives, and dried at 50°C in a drying chamber. The dried extract is finally ground and sieved for an adjustment of the particle size. The yield of native extract is about 35% and the analysis shows water contents of less than 5% and a complete removal of the essential oil (Estragol, Methyleugenol).

The herein disclosed and described water-soluble Russian Tarragon extracts and products derived therefore help to clear glucose from the blood, reduce post-prandial glucose peaks and helps reducing undesired glycoxylation reaction with vital molecular body structures or metabolites in the blood serum or tissues.

Similarly related, such administration of the said extracts or products can also be used for the purpose of normalizing blood glucose levels. The material can be administered as disclosed and described Russian Tarragon extracts or in combinations with or without further anti-diabetic compounds and for instance with ergogenic compounds, e.g. creatine and can be administered in a variety of product forms including capsules, tablets, powdered beverages, bars, gels or drinks.

The invention also relates to the use of water-soluble plant extracts from Russian Tarragon (Artemisia dracunculus var. inodora) and ergogenic nutrients (e.g. creatine and/or carnitine and/or their derivatives) for the enhancement of cellular glucose uptake into major tissues for the purpose to optimized blood glucose levels, which involves the supplementary, prophylactic or therapeutic use, in particular of a water-soluble extract of Russian Tarragon in preferred daily dose of 10 mg to 20,000 mg. The disclosed tarragon extracts and their combinations of further favorable components are not restricted to any particular form of application, which makes them all the more suitable for the different application areas.

Disclosed herein is: (a) a aqueous extract of Russian Tarragon (Artemisia dracunculus var inodora), combinations comprising said tarragon extract with formulation agents or constituents, or an extract fraction thereof or a derivative of the extract thereof; and (b) methods of normalizing elevated blood glucose levels that may occur under conditions of metabolic syndrome, pre-diabetes and diabetes Type 2 in animals and humans, whereas the administration of said composition as dietary supplement, food or drinks preparation, or in pharmaceutical delivery form is possible.

It is a still further object of the invention to provide a method and a dietary supplement that achieves these objects when administered in physiologically acceptable amounts in preferred daily dose of 20 to 5000 mg divided in several (2-5) servings per day.

Also disclosed herein are methods of losing weight and reducing body fat comprising administration of said composition.

Accordingly, it is also an object of the invention to provide a method and a dietary supplement which will promote weight loss and body fat reduction.

Other objectives, advantages and features of the invention will become apparent from the given detailed description, and from the claims.

The disclosed and described invention is a aqueous extract of Russian Tarragon (Artemisia dracunculus var. inodora), combinations comprising the said tarragon extract with formulation agents or constituents. The administration of said composition can also be used for the purpose of enhancing nutrient, e.g. creatine and/or carnitine transport for purposes of athletic performance and improvement of Body Composition-Index (BCI) while controlling bodyweight and body fat levels, and therefore improves the body composition, increases wellness and mental and physical performance during sports, in illness conditions or are circumstance of special needs.

A further embodiment of the invention is a dietary supplement, a food, a beverage or a pharmaceutical product comprising the extract of the invention.

It may additional comprise carbohydrates, like dextrose, maltose, maltodextrin and trehalose, formulation aids, like dissolution enhancer, binder and other auxiliaries, minerals, like Magnesium and Calcium, trace elements, like Vanadium, Chromium, Zinc, methylxanthines, like caffeine, theobromine and theophylline, free amino acids, like taurine, glutamine, citrulline, leucine, glycine, arginine, alanine, or salts of derivatives thereof, vitamins, like vitamin A, C, E, vitamin derivatives, herbs and botanical extracts with or without glucose-level-modifying effect, as well as lactic acid buffering agent, like (sodium) bicarbonate, citrates, phosphates, carnosine, beta-alanine, and mixtures thereof.

Suitable application form comprises powder, capsules, tablets, effervescent tablets, powdered beverages, bars, gels or drinks, pharmaceutical delivery systems.

The product of the invention can be used for regulating, controlling, normalizing elevated blood glucose levels that may occur under conditions of metabolic syndrome, pre-diabetes and diabetes Type 2 in animals and humans, for the prevention and treatment of metabolic diseases, including hyperlipidemia, for improvement of body-composition index (BCI) and for the reduction of body mass, and preferable the reduction of body fat in athletes, sport people, elderly people or overweight and ill patients.

Further embodiment of the invention is the use of Russian tarragon (Artemisia dracunculus var. inodora), an extract of Tarragon or a derivative thereof to increase uptake of ergogenic substances.

Glucose metabolism in humans is often linked to inflammatory diseases of individual organs. So it is beneficial to find a general antidiabetic active extract combined with an anti-inflammatoric potential.

A hallmark of inflammation is the secretion of huge amounts of proinflammatory immune mediators such as prostaglandin E₂, and cytokines, e.g. tumor necrosis factor. Local secretion of cytokines and activation of immunocompetent cells in the microenvironment are followed by systemic reaction.

Primary human monocytes are a well established cell model to investigate the effects of compounds and plant extracts on their capability to inhibit cytokine release.

The aqueous Tarragon extract of the invention shows a dose dependent inhibition of the TNFα release. The strongest inhibition of about 80% at the concentration of 300 µg/ml has been found. This demonstrates a significant anti-inflammatoric activity.

The following examples are used to explain the invention in more details without limiting the scope.
Figure 1 shows the influence of the plant variety on blood glucose levels according to example 13.
Figure 2 shows the influence of the extraction solvents on blood glucose levels according to example 13.
Figure 3 shows the glucose challenge blood glucose levels AUC (area under the curve) 0 to 180 minutes according to example 11.

### Example 1 (Comparative example)

### Preparation of a hydroethanolic extract of Russian Tarragon:

1 kg of the raw material of Russian Tarragon was extracted twice with 6 L of 60% ethanol (V/V) at 50°C. After cooling of the eluate over night, the solutions were filtered through paper filters and the solvent evaporated by means of a rotatory evaporator. The obtained dense extracts was mixed with 30% of microcrystalline cellulose as suitable carrier, and dried at 50°C in a vacuum drying chamber. The dried extract was finally ground and sieved for an adjustment of the particle size. The yield of native extract is about 20% and the analysis showed water contents of less than 2% and an almost complete removal of the essential oil (Estragol, Methyleugenol) of less than 3 ppm.

### Example 2 (Comparative example)

### Preparation of aqueous extracts of French Tarragon:

1 kg of the raw material of French Tarragon was extracted twice with 6 L water at 80°C. After cooling of the eluate over night, the solutions were separated from the drug by filtration through paper filters and the solvent evaporated by means of a rotatory evaporator. The obtained dense extracts was mixed with 30% of maltodextrin as suitable carrier, and dried at 50°C in a vacuum drying chamber. The dried extract was finally ground and sieved for an adjustment of the particle size. The yield of native extract is about 28% and the analysis showed water contents of less than 4% and a complete removal of the essential oil (Estragol, Methyleugenol).

### Example 3

### Preparation of aqueous extracts of Russian Tarragon:

1 kg of the raw material of Russian Tarragon was extracted twice with 6 L water at 80°C. After cooling of the eluate over night, the solutions were separated from the drug by filtration through paper filters and the solvent evaporated by means of a rotatory evaporator. The obtained dense extracts was mixed with 30% of maltodextrin as suitable carrier, and dried at 50°C in a vacuum drying chamber. The dried extract was finally ground and sieved for an adjustment of the particle size. The yield of native extract is about 32% and the analysis showed water contents of less than 4% and a complete removal of the essential oil (Estragol, Methyleugenol).

### Example 4:

### Fractionation of aqueous extracts of Russian Tarragon by means of membrane techniques:

300 g extract of example 2, diluted in demineralized water to 20% (w/w), was divided by ultrafiltration with a middle cut-off of about 100 kDa in two fractions (retentate and permeate). Separation results in a volume distribution of retentate : permeate of 1 : 12 respectively a mass distribution of residual dry mass of 1 : 9. The active principle cumulates in permeate.

### Example 5

### Fractionation of aqueous extracts of Russian Tarragon by means of absorption column techniques:

200 g extract analogous to example 2, diluted in demineralized water to 20% (w/w), was given onto adsorptive resin (Amberlite® XAD7HP). The active priciple remains in passing aqueous phase, with a yield of about 95%, whereas organic middle-polar constituents were removed by the column. The active principle cumulates in this purified aqueous extract.

### Example 6

### Alternative removal of essential oil before extraction:

1 kg of the raw material of Russian Tarragon was damped by superheated steam for about 1 hour and afterwards extracted twice with 6 L water at 25°C. The eluates were separated from the drug by filtration through paper filters and the solvent evaporated by means of a rotatory evaporator. The obtained dense extracts showed a complete removal of the essential oil (Estragol, Methyleugenol).

### Example 7

### Preparation of tablets

| **Ingredient** | **Amount per tablet** | |
|---|---|---|
| Russian Tarragon aqueous extract | 225 | mg |
| Zinc (as sulfate, chloride or pyruvate) | 15 | mg |
| Calcium phosphate | 165 | mg |
| Methylcellulose | 150 | mg |
| Stearic acid | 24 | mg |
| Magnesium stearate | 7 | mg |
| Silicon dioxide | 10 | mg |
| **TOTAL** | **596** | **mg** |

The recommended daily dose is 3-6 tablets.

### Production procedure

1. All the active substances and adjuvants are sieved through a sieve with a mesh size of 1.0 mm.
2. The tarragon extract and the excipients are weighed and introduced into a mixer. The blend is mixed, using schedules as known by galenic qualified staff. The homogeneity of the mixture is checked visually.
3. The tablets are compressed directly from the mixture.

### Example 8

### Preparation of Soft-Gelatin Capsule

### Formulation:

| **Ingredient** | **Amount per tablet** | |
|---|---|---|
| Russian Tarragon aqueous extract | 350 mg | |
| Medium Chain Triglycerides | 125 mg | |
| **TOTAL** | **475 mg** | |

Filled in Soft-Gelatin Capsule. The recommended daily dose is 3-6 capsules.

### Production Procedure

1. All the active substances, adjuvants, and the diluent are weighed and introduced into a mixer. The blend is mixed for 15 minutes. The homogeneity of the obtained paste is checked visually.
2. The soft-gel capsule can be filled using standard industrial equipment.

### Example 9

### Preparation of chewable tablets

For the preparation of the tablets a commercial available chewable matrix based on Mannitol or Sorbitol, starch (or its derivatives), sweetener, and other excipients were used.

### Formulation:

| **Ingredient** | **Amount per tablet** | |
|---|---|---|
| Russian Tarragon aqueous extract | 175 | mg |
| Caffeine | 75 | mg |
| Chewable matrix | 1500 | mg |
| Sodium bicarbonate | 50 | mg |
| Flavors | 90 | mg |
| Magnesium stearate | 25 | mg |
| Silicon dioxide | 10 | mg |
| **TOTAL** | **1925** | **mg** |

The recommended daily dose is 3-4 chewable tablets.

### Production procedure

1. All the active substances and adjuvants are sieved through a sieve with a mesh size of 1.0 mm.
2. The Tarragon extract and the excipients are weighed and introduced into a mixer. The blend is mixed for 30 minutes. The homogeneity of the mixture is checked visually.
3. The tablets are compressed directly from the mixture.

The daily dose is one chewable tablets three times a day.

### Example 10

### Preparation of effervescent tablets

### Formulation :

| **Ingredient** | **Amount per tablet** | |
|---|---|---|
| Russian Tarragon aqueous extract | 150 | mg |
| Caffeine | 75 | mg |
| Anhydrous citric acid | 1700 | mg |
| Sodium hydrogen carbonate | 1000 | mg |
| Polyethylene glycol 2000 | 500 | mg |
| Sweetener | 70 | mq |
| Orange flavor | 90 | mg |
| Magnesium stearate | 20 | mg |
| **TOTAL** | **3605** | **mg** |

The recommended daily dose is 2-4 effervescent tablets dissolved each in 300 ml of water.

### Production procedure

1. All the active substances and adjuvants are sieved through a sieve with a mesh size of 1.0 mm.
2. All ingredients are weighed and introduced into a mixer. The mixture is mixed using schedules as known by galenic qualified staff. The homogeneity of the mixture is checked visually.
3. The tablets are compressed directly from the mixture.

### Example 11

### Preparation of nutritional bars

Formula of the bar filling: 1/2 cup Sugar, 5 tb Cornstarch, 3 tb Brown sugar, 1/4 ts Salt, 3 c Milk, 3 Egg yolks,beaten 1 ts Vanilla, 8 oz Chocolatebar, and 4750 mg Russian tarragon water extract, 25 grams of micronized creatine monohydrate, 1000 mg vitamin blend (e.g. Multi 10, Roche, RDA=200mg), 1200 mg Calcium as Carbonate, 450 mg Magnesium as Carbonate, and 50 mg Zinc as Sulfate.

### Production Procedure

Combine all but vanilla and chocolate bar in a saucepan. Stir constantly until mixture boils; boil and stir 1 minute. Remove from heat; add vanilla and chocolate bar, broken into pieces. Stir until chocolate is completely melted. Add the Russian Tarragon extracts, creatine, vitamins, and minerals. Pour into bowl and press plastic wrap directly on surface; cool. Yields about 4 cups filling or about 10 bars of 100 grams each.

The recommended daily dose is two to four nutritional bars.

### Example 12

### Preparation of refreshing and energizing powder drink formulation

### One serving size of this drink contains:

| | |
|---|---|
| Russian Tarragon water extract | 350mg |
| Creatine monohydrate | 500 mg |
| L-Camitine L-tartrate | 1000 mg |
| Amino acids (protein hydrolysate) | 7.5 g |
| Carbohydrates (Maltodextrin) | 7.5 g |
| weetener | 70 mg |
| Orange flavor | 90 mg |
| Vitamin B1 | 0.7 mg |
| Vitamin B2 | 0.8 mg |
| Vitamin B6 | 1 mg |
| Vitamin B12 | 0.5 µg |
| Vitamin C | 30 mg |
| Vitamin E | 5 mg |
| Niacinamide | 9 mg |
| Folsaure | 100 µg |
| Biotin | 75 µg |
| Pantothenic acid | 3 mg |
| calcium | 120 mq |
| Magnesium | 45 mg |
| Zinc | 5 mg |

### Production procedure

For the production on technical scale the above shown quantities of the blend should be multi-fold with a factor up to 10000 or even higher to obtain 200 kg+ batches.
1 All the active substances and adjuvants are sieved through a sieve with a mesh size of 1.0 mm.
2. The aqueous Russian Tarragon extract, creatine monohydrate and creatine pyruavte, amino acids, maltodextrin, and the other components are weighed and introduced into a mixer. The mixture is mixed for 45 minutes. The homogeneity of the mixture is checked visually.
3. The powder tablets are filled in powder bottles directly from the mixture.

*A daily dose is twice of the single dosage shown above. This drink powder needs to be stirred in about 400 ml of water prior use.*

### Example 13

### Investigation on the Russian Tarragon extracts in an animal model

### Animal model for a Glucose challenge test

A common way of testing for an effect of extracts or new chemical entities on blood glucose levels is the glucose challenge test (verspohl, E. J.: Recommended testing in diabetes research. Planta Med 68 (7): 581-90, 2002) in which rats are given the extract, control, or a known antidiabetic substance with (challenge) and without (basal) an intraperitoneal (i.p.) dose of glucose. The extracts are given orally 30 min. before the glucose challenge. Blood samples are taken sublingual at time points 0, 15, 30, 60, and 120 minutes for the glucose challenge or 0, 30, 60, 120, and 180 minutes for basal blood glucose levels. This sampling scheme ensures to monitor both effects of extracts on basal blood glucose levels over a longer time period and the blood glucose levels after glucose challenge closely enough.

### Animals

Male non-fasted Wistar rats weighing 250-300 g were purchased from Harlan (Indianapolis, IN, U.S.A). The non-fasted condition was chosen to account for a more physiological situation, but increases variability of blood glucose levels. Rats were housed in cages of 2 at 20 ± 1 °C in a 12-h light/dark cycle. Tap water and food pellets were available ad libitum. Groups of 6 rats were randomly assigned to the 12 different treatment groups. All experiments were carried out in a quiet room between 9:00 a.m. and 2:00 p.m. All animals were housed and all experiments performed according to the policies and guidelines of the Institutional Animal Care and Use Committee (IACUC) of the University of Florida, Gainesville, U.S.A.

### Drugs

French or Russian Tarragon extracts were prepared by dissolving the various extracts (Russian Tarragon extracts according to example 1 (ethanolic) and 3 (water), French Tarragon extract according example 2 (water) 6 mg/kg each); in 5 ml deionized water with 0.5% propylene glycol to form a suspension. All solutions were prepared freshly on test days. All animals were brought to the testing room at least 30 minutes prior to testing and remained in the same room throughout the test. Animals were orally treated with control (vehicle) or the extracts. Glucose (Sigma-Aldrich) was dissolved (using sonication) in 0.9% saline solution in a concentration of 2 g/5 mL and given i.p. 30 minutes after the oral treatment.

### Blood glucose evaluation

Blood was drawn at the appropriate time points from the sublingual vein after a short halothane anesthesia and stored at 4 °C with addition of heparin. Samples were centrifuged at 8600 rpm for 10 minutes. The supernatant plasma was taken and analyzed using an autoanalyzer (Merck, Darmstadt). Analytical plasma controls and matrix blanks were used to guarantee accurate results within the specified limits.

### Statistics and calculations

Both percent and AUC data were analyzed by one-way ANOVA and Student-Newman-Keuls Multiple Comparison Test using Graphpad 4.0 Software, San Diego, USA. The AUC (area under the curve) was calculated using the trapezoidal rule without extensions beyond the last time point measured.

### Results and discussion

### Basal blood glucose levels

One-way ANOVA statistical analysis followed by Student-Newman-Keuls posthoc test revealed a significant effect on basal blood glucose levels for the known antidiabetic glyburide and sitagliptin (used as reference materials) but no effect for the Tarragon extracts. The time point comparison was not significant for any of the extracts, whereas glyburide and sitagliptin lowered the basal blood glucose levels significantly. The finding of no effect of the extracts of the basal blood glucose levels can be interpreted as crucial positive safety aspect related to missing of a hypoglycemia status after oral administration of the said extract by fasting individuals.

### 3.2 Glucose challenge blood glucose levels

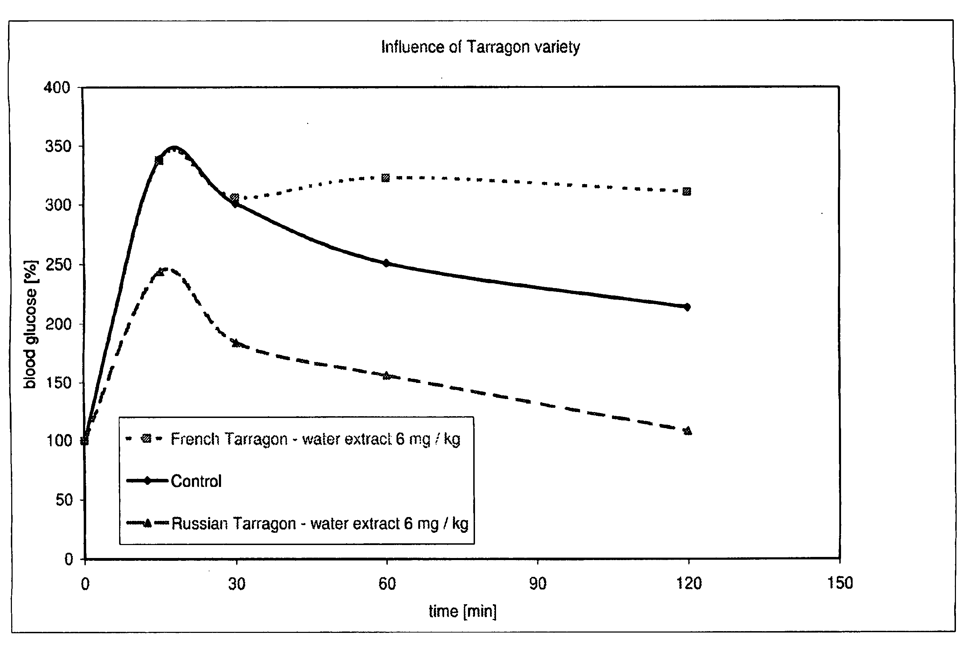

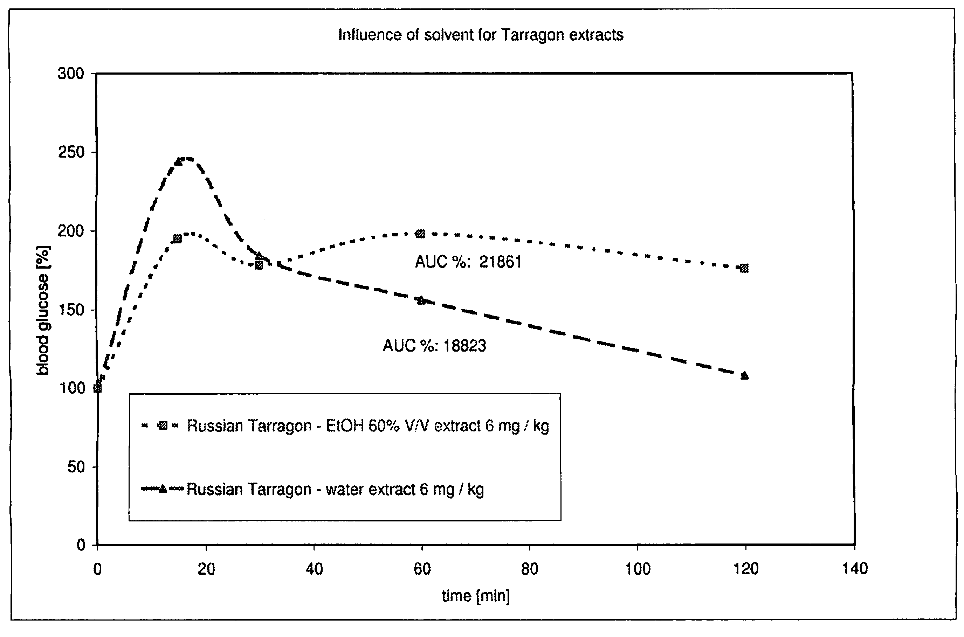

The statistical analysis using a one-way ANOVA test followed by Student-Newman-Keuls multiple comparison concluded that glyburide, sitagliptin, and extract 3 (Russian Tarragon, water) had a significant effect on lowering the blood glucose levels following the i.p. glucose challenge. Extract 2 (French Tarragon, water) could not show any positiv effect on blood glucose level (Figure 1); extract 1 (Russian Tarragon, 60% ethanolic) lowered the blood glucose level also, but did not reach significance in this animal test due to high AUC (Figure 2).

The Student-Newman-Keuls multiple comparison allows for pair wise comparisons (also called range or multiple-step statistics) as it has different critical values for each pair wise comparison depending on the difference of the means. This method presents with a higher power compared to the Tukey's Test (a one-step procedure), which uses only one critical value for pair wise comparisons without adjusting for the difference in means. Both tests are used as post-hoc tests to compare more than two treatment means (if only two treatments are compared, the Student t-test would be the appropriate test to be used).

The comparison of the single time points in this experimental setting (5 treatment groups and physiological non-fasted animals) presents with the problem of significant variation that might mask a potential blood glucose lowering effect. This variability can be accounted for in part by comparing the percent change in blood glucose levels over time in relation to the initial levels at time point zero.

In conclusion, these results show an effect on blood glucose levels after glucose challenge for the known antidiabetic drugs glyburide, sitagliptin, for the evaluated extracts 1 and 3, although extract 1 (ethanolic) did not reach significance in the performed test.

### Example 14

### Reducing TNF-a release

Human primary monocytes were prepared from buffy coats of healthy human blood donors following a standardized procedure. Cells were seeded in 24-well-plates for ELISA measurements.

### Monocytic cell treatment and measurement of TNFalpha

Monocytes were stimulated with LPS (10ng/ml) at 37°C and 5 % CO₂ for 24 h. The extracts were added 30 min before LPS treatment to test if they can prevent the LPS-induced effects. The following concentrations were tested: 50, 100, 300, 400, and 500 µg/ml. After 24 h, supernatants were removed, centrifugated and investigated for TNFalpha concentrations in ELISAs/EIAs using manufactorer's protocol (Biotrend, Germany; Immunotools, Germany).

The results demonstrate a clear dose depending inhibition of the TNF-alpha release, which is related to an anti-inflammatory activity. IC50 value is about 120 µg per ml.

## Claims

1. An extract obtainable by a method comprising the steps
- extracting Russian Tarragon (Artemisia dracunculus var. inodora) with water
- wherein the extraction medium is at least partially removed to get a concentrated extract or completely removed to get a dry extract.

2. The extract of claim 1 wherein extracting is conducted for 1 hour to 40 hours.

3. The extract of any one of claims 1 or 2, wherein further purification steps are conducted, preferably ultrafiltration or absorption steps.

4. The extract of any one of claims 1 to 3 wherein the amount of essential oils, e.g. estragol and methyleugenol are below 50 ppm.

5. A method for the production of an extract of Russian Tarragon (Artemisia dracunculus var. inodora) comprising the step of
- extracting Russian Tarragon (Artemisia dracunculus var. inodora) with water
- wherein the extraction medium is at least partially removed to get a concentrated extract or completely removed to get a dry extract.

6. The method of claim 5, wherein extraction is conducted at temperatures from 20°C to 95°C, preferably from 50 to 80°C.

7. The method of claim 5 or 6, wherein further purification steps are conducted.

8. A dietary supplement, food, beverage or pharmaceutical product comprising the extract of any one of claims 1 to 4.

9. The dietary supplement, food, beverage or pharmaceuticals product of claim 8, further comprising carbohydrates, like dextrose, maltose, maltodextrin and trehalose, formulation aids, like dissolution enhancer, binder and other auxiliaries, minerals, like Magnesium and Calcium, trace elements, like Vanadium, Chromium, Zinc, methylxanthines, like caffeine, theobromine and theophylline, free amino acids, like taurine, glutamine, citrulline, leucine, glycine, arginine, alanine, or salts or derivatives thereof, vitamins, like vitamin A, C, E, vitamin derivatives, herbs and botanical extracts with or without glucose-level-modifying effect, as well as lactic acid buffering agent, like (sodium) bicarbonate, citrates, phosphates, carnosine, beta-alanine, and mixtures thereof.

10. The dietary supplement, food, beverage or pharmaceuticals product of claim 8 or 9 in the form of a powder, of capsules, tablets, effervescent tablets, powdered beverages, bars, gels or drinks, pharmaceutical delivery systems.

11. Russian Tarragon (Artemisia dracunculus var. inodora) extract of any one of claims 1 to 4 for use in regulating, controlling, normalizing elevated blood glucose levels that may occur under conditions of metabolic syndrome, pre-diabetes and diabetes Type 2 in animals and humans, for the prevention and treatment of metabolic diseases, including hyperlipidemia, for improvement of body-composition index (BCI) and for the reduction of body mass, and the reduction of body fat in athletes, sport people, elderly people or overweight and ill patients, as an anti-inflammatory drug in medical preparations or dietary supplements in joint inflammation or arthritis.

12. Water-soluble extract of Russian Tarragon (Artemisia dracunculus var. inodora) of any one of claims 1 to 4 for use to increase uptake of glucose in major tissues and thus lowering of elevated blood glucose levels, especially of post-prandial glucose levels.

13. Russian Tarragon extract of any one of claims 1 to 4 for use in the treatment or prevention of metabolic syndrome, and/or treatment or prevention of pre-diabetes.

14. Russian Tarragon (Artemisia dracunculus var. inodora) extract of any one of claims 1 to 4 or a fraction thereof for use in treatment or prevention of diabetes Type 2.

15. Extract of any one of claims 1 to 4 for use as anti-inflammatory drug in medical preparations or dietary supplements in joint inflammation or arthritis.

## Patentansprüche

1. Extrakt, erhältlich nach einem Verfahren, das die folgenden Schritte umfasst:
- Extrahieren von Russischem Estragon (*Artemisia dracunculus* var. *inodora*) mit Wasser;
- wobei das Extraktionsmedium wenigstens teilweise unter Bildung eines konzentrierten Extrakts oder vollständig unter Bildung eines Trockenextrakts entfernt wird.

2. Extrakt gemäß Anspruch 1, wobei das Extrahieren während 1 Stunde bis 40 Stunden durchgeführt wird.

3. Extrakt gemäß einem der Ansprüche 1 oder 2, wobei weitere Reinigungsschritte durchgeführt werden, vorzugsweise Ultrafiltrations- oder Absorptionsschritte.

4. Extrakt gemäß einem der Ansprüche 1 bis 3, wobei die Menge der ätherischen Öle, zum Beispiel Estragol und Methyleugenol, unter 50 ppm liegt.

5. Verfahren zur Herstellung eines Extrakts von Russischem Estragon (*Artemisia dracunculus* var. *inodora*), umfassend die Schritte:
- Extrahieren von Russischem Estragon (*Artemisia dracunculus* var. *inodora*) mit Wasser;
- wobei das Extraktionsmedium wenigstens teilweise unter Bildung eines konzentrierten Extrakts oder vollständig unter Bildung eines Trockenextrakts entfernt wird.

6. Verfahren gemäß Anspruch 5, wobei die Extraktion bei Temperaturen von 20°C bis 95 °C, vorzugsweise 50 bis 80°C, durchgeführt wird.

7. Verfahren gemäß Anspruch 5 oder 6, wobei weitere Reinigungsschritte durchgeführt werden.

8. Nahrungsergänzungsmittel, Nahrungsmittel, Getränk oder pharmazeutisches Produkt, das den Extrakt gemäß einem der Ansprüche 1 bis 4 umfasst.

9. Nahrungsergänzungsmittel, Nahrungsmittel, Getränk oder pharmazeutisches Produkt gemäß Anspruch 8, das weiterhin Kohlenhydrate, wie Dextrose, Maltose, Maltodextrin und Trehalose, Formulierungshilfsmittel, wie Auflösungsverstärker, Bindemittel und andere Hilfsstoffe, Mineralien, wie Magnesium und Calcium, Spurenelemente, wie Vanadium, Chrom, Zink, Methylxanthine, wie Coffein, Theobromin und Theophyllin, freie Aminosäuren, wie Taurin, Glutamin, Citrullin, Leucin, Glycin, Arginin, Alanin oder Salze oder Derivate davon, Vitamine, wie Vitamin A, C, E, Vitaminderivate, Kräuter und Pflanzenextrakte mit oder ohne den Glucosespiegel modifizierende Wirkung sowie Milchsäure-Puffermittel, wie (Natrium)hydrogencarbonat, Citrate, Phosphate, Carnosin, beta-Alanin und Gemische davon, umfasst.

10. Nahrungsergänzungsmittel, Nahrungsmittel, Getränk oder pharmazeutisches Produkt gemäß Anspruch 8 oder 9 in Form eines Pulvers, von Kapseln, Tabletten, Brausetabletten, Getränkepulvern, Riegeln, Gelen oder Getränken, pharmazeutischen Abgabesystemen.

11. Extrakt von Russischem Estragon (*Artemisia dracunculus* var. *inodora*) gemäß einem der Ansprüche 1 bis 4 zur Verwendung bei der Regulierung, Senkung, Normalisierung erhöhter Blutglucosewerte, die unter Bedingungen eines metabolischen Syndroms, Prädiabetes und Diabetes Typ 2 bei Tieren und Menschen auftreten können, zur Prävention und Behandlung von Stoffwechselerkrankungen einschließlich Hyperlipidämie, zur Verbesserung des Körper-Zusammensetzung-Index (BCI) und zur Reduktion der Körpermasse sowie zur Reduktion von Körperfett bei Athleten, Sportlern, älteren Menschen oder übergewichtigen und kranken Patienten sowie als entzündungshemmender Wirkstoff in medizinischen Präparaten oder Nahrungsergänzungsmitteln bei Gelenkentzündung oder Arthritis.

12. Wasserlöslicher Extrakt von Russischem Estragon (*Artemisia dracunculus* var. *inodora*) gemäß einem der Ansprüche 1 bis 4 zur Verwendung zur Erhöhung der Aufnahme von Glucose in größeren Geweben und somit Senkung von erhöhten Blutglucosewerten, insbesondere von postprandialen Glucosewerten.

13. Extrakt von Russischem Estragon gemäß einem der Ansprüche 1 bis 4 zur Verwendung bei der Behandlung oder Prävention von metabolischem Syndrom und/oder bei der Behandlung oder Prävention von Prädiabetes.

14. Extrakt von Russischem Estragon (*Artemisia dracunculus* var. *inodora*) gemäß einem der Ansprüche 1 bis 4 oder eine Fraktion davon zur Verwendung bei der Behandlung oder Prävention von Diabetes Typ 2.

15. Extrakt gemäß einem der Ansprüche 1 bis 4 zur Verwendung als entzündungshemmender Wirkstoff in medizinischen Präparaten oder Nahrungsergänzungsmitteln bei Gelenkentzündung oder Arthritis.

## Revendications

1. Extrait pouvant être obtenu par un procédé comprenant les étapes suivantes
- extraction d'estragon de Russie (Artemisia dracunculus var. inodora) avec de l'eau
- dans lequel le milieu d'extraction est au moins partiellement éliminé pour obtenir un extrait concentré ou complètement éliminé pour obtenir un extrait sec.

2. Extrait selon la revendication 1, dans lequel l'extraction est conduite pendant 1 heure à 40 heures.

3. Extrait selon l'une quelconque des revendications 1 ou 2, dans lequel des étapes de purification supplémentaires sont conduites, de préférence des étapes d'ultrafiltration ou d'absorption.

4. Extrait selon l'une quelconque des revendications 1 à 3, dans lequel la quantité d'huiles essentielles, par exemple l'estragol et le méthyleugénol, est en dessous de 50 ppm.

5. Procédé de production d'un extrait d'estragon de Russie (Artemisia dracunculus var. inodora) comprenant l'étape suivante
- extraction d'estragon de Russie (Artemisia dracunculus var. inodora) avec de l'eau
- dans lequel le milieu d'extraction est au moins partiellement éliminé pour obtenir un extrait concentré ou complètement éliminé pour obtenir un extrait sec.

6. Procédé selon la revendication 5, dans lequel l'extraction est conduite à des températures de 20 °C à 95°C, de préférence de 50 à 80 °C.

7. Procédé selon la revendication 5 ou 6, dans lequel des étapes de purification supplémentaires sont conduites.

8. Supplément diététique, aliment, boisson ou produit pharmaceutique comprenant l'extrait selon l'une quelconque des revendications 1 à 4.

9. Supplément diététique, aliment, boisson ou produit pharmaceutique selon la revendication 8, comprenant en outre des glucides, tels que le dextrose, le maltose, la maltodextrine et le tréhalose, des aides de formulation, telles qu'un amplificateur de dissolution, un liant et d'autres auxiliaires, des minéraux, tels que le magnésium et le calcium, des oligoéléments, tels que le vanadium, le chrome, le zinc, des méthylxanthines, telles que la caféine, la théobromine et la théophylline, les acides aminés libres, tels que la taurine, la glutamine, la citrulline, la leucine, la glycine, l'arginine, l'alanine, ou des sels de dérivés de ceux-ci, des vitamines, telles que la vitamine A, C, E, des dérivés de vitamine, des herbes et des extraits botaniques avec ou sans effet de modification du taux de glucose, ainsi qu'un agent tampon d'acide lactique, tel que le bicarbonate (de sodium), des citrates, des phosphates, la carnosine, la bêta-alanine, et des mélanges de ceux-ci.

10. Supplément diététique, aliment, boisson ou produit pharmaceutique selon la revendication 8 ou 9, sous la forme d'une poudre, de capsules, de comprimés, de comprimés effervescents, de boissons en poudre, de barres, de gels ou de boissons, de systèmes d'administration pharmaceutiques.

11. Extrait d'estragon de Russie (Artemisia dracunculus var. inodora) selon l'une quelconque des revendications 1 à 4, destiné à être utilisé dans la régulation, le contrôle, la normalisation des taux de glycémie élevés qui peuvent se produire dans des affections du syndrome métabolique, du prédiabète et du diabète de type 2 chez les animaux et les humains, pour la prévention et le traitement de maladies métaboliques, comprenant l'hyperlipidémie, pour l'amélioration de l'indice de composition corporelle (CCI) et pour la réduction de la masse corporelle, et la réduction des corps gras chez des athlètes, des sportifs, des personnes âgées ou des patients en surpoids et malades, en tant que médicament anti-inflammatoire dans des préparations médicales ou des suppléments diététiques dans l'inflammation articulaire ou l'arthrite.

12. Extrait d'estragon de Russie (Artemisia dracunculus var. inodora) soluble dans l'eau selon l'une quelconque des revendications 1 à 4, destiné à être utilisé pour augmenter l'absorption de glucose dans des tissus principaux et abaisser de ce fait les taux de glycémie élevés, notamment les taux de glucose postprandiaux.

13. Extrait d'estragon de Russie (Artemisia dracunculus var. inodora) selon l'une quelconque des revendications 1 à 4, destiné à être utilisé dans le traitement ou la prévention d'un syndrome métabolique, et/ou le traitement ou la prévention du prédiabète.

14. Extrait d'estragon de Russie (Artemisia dracunculus var. inodora) selon l'une quelconque des revendications 1 à 4, ou fraction de celui-ci, destiné à être utilisé dans le traitement ou la prévention du diabète de type 2.

15. Extrait selon l'une quelconque des revendications 1 à 4, destiné à être utilisé en tant que médicament anti-inflammatoire dans des préparations médicales ou des suppléments diététiques dans l'inflammation articulaire ou l'arthrite.
